# EUROPEAN PATENT SPECIFICATION

(11) **EP 4 004 192 B1**
(45) Date of publication and mention of the grant of the patent: **23.10.2024**
(21) Application number: 20739699.5
(22) Date of filing: 16.07.2020
(51) Int. Cl.: C12N 5/078, C12N 5/0789, G01N 33/50

(54) **ASSAY FOR IDENTIFING COLONY-FORMING CELLS**
TEST ZUR IDENTIFIZIERUNG VON KOLONIEBILDENDEN ZELLEN
ANALYSE D'IDENTIFICATION DE CELLULES FORMANT DES COLONIES

(30) Priority: 24.07.2019 EP 19188003
(43) Date of publication of application: 01.06.2022
(73) Proprietor: Miltenyi Biotec B.V. & Co. KG, 51429 Bergisch Gladbach (DE)
(72) Inventor: BISSELS, Ute, 50823 Köln (DE); BOSIO, Andreas, 51429 Bergisch Gladbach (DE); ROCKEL, Thomas, 51429 Bergisch Gladbach (DE); VÖLKEL, Andrea, 51429 Bergisch Gladbach (DE)
(86) International application number: PCT/EP2020/070076
(87) International publication number: WO 2021/013669

(56) References cited:
- WO-A1-96/15229
- WO-A2-03/050251
- WO-A2-2004/018996
- US-A1- 2004 110 243
- US-A1- 2004 110 286
- US-A1- 2007 059 778
- GRIGORIOS GEORGOLOPOULOS ET AL: "Unbiased phenotypic identification of functionally distinct hematopoietic progenitors", JOURNAL OF BIOLOGICAL RESEARCH-THESSALONIKI, vol. 26, no. 1, 18 July 2019 (2019-07-18), XP055647357, DOI: 10.1186/s40709-019-0097-7

## Description

### BACKGROUND

The present invention is directed to an assay and a process for detection or identification of colony forming cells and discrimination of colonies derived from those cells, especially hematopoietic stem cells (HSCs).

The hematopoietic stem cells (HSCs) like every stem cell, can self-renew and simultaneously give rise to hematopoietic progenitors which will subsequently and eventually differentiate towards either the lymphoid lineage, giving rise to T-, B- and NK-cells and towards the myeloid lineage, giving rise to erythrocytes, platelets, granulocytes, macrophages etc. After a long time of experimentation it became clear that in humans the cells possessing the ability to differentiate to the several multilineage progenitors was the CD34+ cell population. Indeed, this was confirmed both in vivo in mouse models as well as in the hematopoietic stem cell transplantation (HSCT) setting. In vitro the most reliable, easy and long-standing assay has been the colony forming cell (CFC) assay, also referred to as the methylcellulose assay. The assay is based on the ability of CD34+ cells to differentiate into distinct colonies which can then be enumerated and characterized.

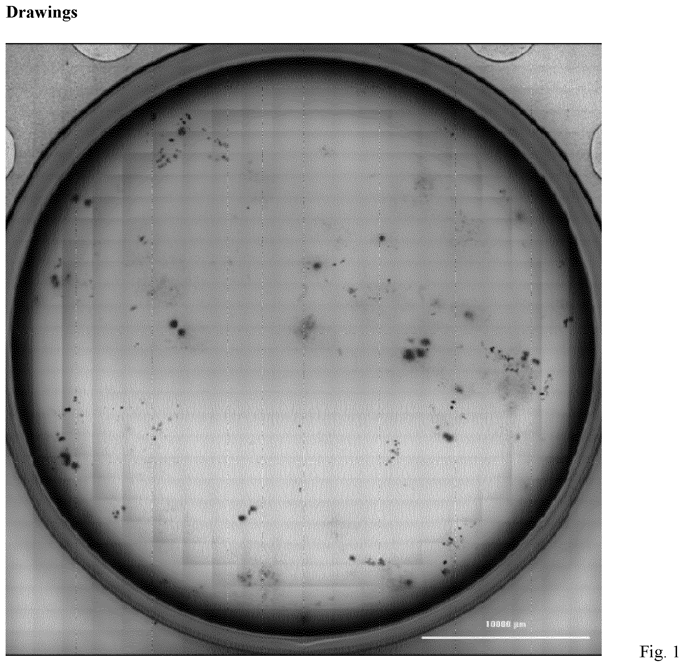

In the classic CFC assay, a certain number of CD34+ cells (usually between 250-500 cells) is plated on a 35mm dish, in 1.1ml of a medium able to support differentiation of the myeloid lineage. This medium consists of IMDM supplemented with cytokines, for example SCF, Flt3 ligand, TPO, IL3 and IL6, etc, fetal bovine serum and methylcellulose to make the medium of high viscosity. The idea behind this is that the cells will not be able to move freely inside the liquid medium, but instead they will remain at a certain position in the methylcellulose semi-solid medium. In this way, after an incubation of approximately 14 days, the 35mm dish will contain many different colonies, each colony deriving, in theory at least, from a single CD34+ cell making possible subsequent measurements of the percentage of CD34+ able to form colonies and of the type of the colony. In that way, colonies derived from different types of progenitor cells are classified and counted based on the number and types of mature cells they contain using morphological and phenotypic criteria after observation using an inverted microscope. Fig. 1 shows a typical result of a CFC colony assay from a 35mm dish. The dots represent different types of colonies.

The colonies formed in the known methylcellulose CFU assays are classified in the following categories: colony-forming unit-erythroid (CFU-E), burst-forming unit-erythroid (BFU-E), colony forming unit macrophage (CFU-M), colony forming unit granulocyte-macrophage (CFU-GM) and colony forming unit granulocyte-erythroid-macrophage-megakaryocyte (CFU-GEMM).

BFU-E contains more than 200 early erythroblasts and are typically found in 3-8 densely packed clusters. CFU-E is smaller compared to the BFU-E and contains 8-200 erythrocyte progenitor cells, typically found in one or two densely packed clusters. Both CFU-E and BFU-E have a dark red/orange to brownish color because of hemoglobin-containing cells. CFU-GEMM presents a compact area that is usually central to a peripheral flat lawn of translucent cells that may be either large or small ("fried egg" appearance).

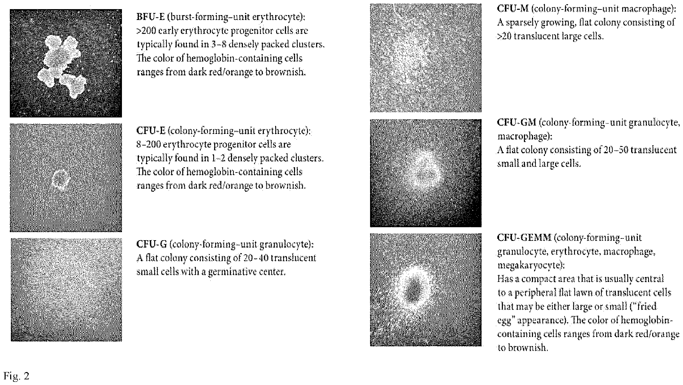

Because CFU-GEMM also contains erythroblasts, depending on the level of hemoglobinization, the color of hemoglobin containing cells can vary from dark red/orange to brownish. CFU-G is usually flat and consists of 20-40 translucent small cells with a germinative center. Finally, CFU-M is a sparsely growing, flat colony consisting of >20 translucent large cells while CFU-GM is also a flat colony consisting of 20-50 translucent small and large cells. A typical result of CFU assay is depicted in Figure 1 where the different type of colonies can be visualized and eventually enumerated based solely on morphological and phenotypic criteria as previously mentioned. Fig. 2 illustrates the different colony types in higher magnification.

Unfortunately, the way colonies are currently counted, by utilizing morphological and phenotypic criteria, can be biased and is largely dependent on the experience of the person who counts them. To determine the degree of variability, we performed several experiments in which we asked from several researchers to count the same 35mm dish which contained colonies after the 14-day incubation period.

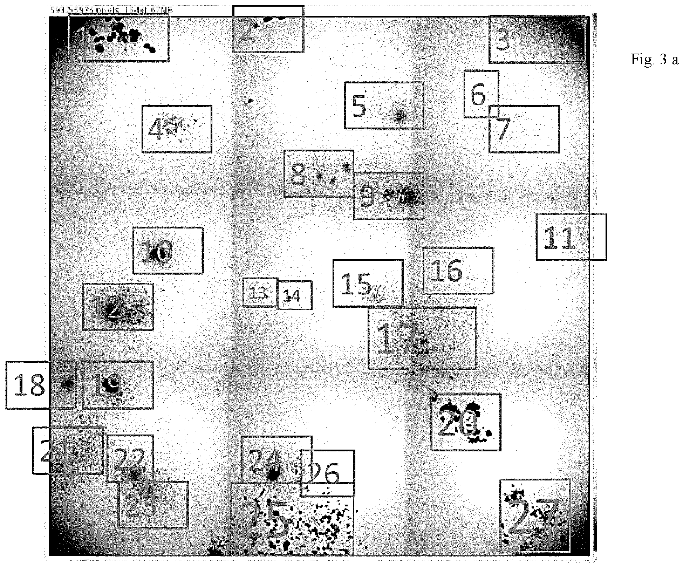

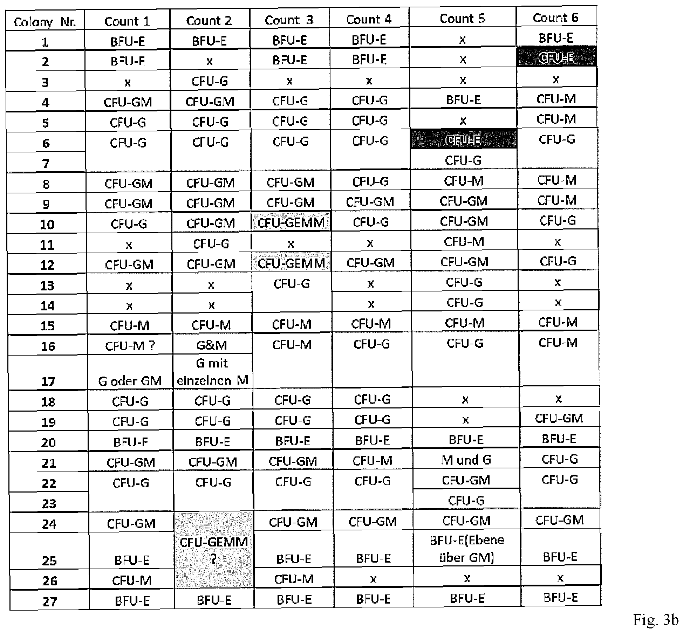

As illustrated in Figure 3 (a and b) from one representative experiment, after enumeration and characterization of the same colonies, the results showed that there was a very high degree of variability in identifying the total number of colonies and assigning the colony to the correct type. More specifically, only 6 out of 27 colonies in total were properly counted and evaluated while for less than a quarter of the colonies there was a clear identification. Obviously, the BFU-E type of colony who is the most easily identifiable colony yielded accurate and reproduceable results. These findings strongly suggest that morphological and phenotypic classification is neither robust nor reproducible.

### SUMMARY

Accordingly, object of the invention is a method for detecting differentiated hematopoietic cells comprising the steps:
a) isolation of undifferentiated hematopoietic stem cells in groups of 1 - 1000 cells on a support
b) proliferating the isolated cells to form cell colonies of differentiated hematopoietic cells by providing cell media comprising a growth factor
c) contacting the cell colonies with one or more marker conjugates comprising at least one detection moiety and at least one antigen recognizing moiety against CD14, CD235a and CD15.
d) detecting the relative amount of differentiated hematopoietic stem cells in a cell colony labelled with the marker conjugates.

Further object of the invention is the use of the method for determining the differentiation status of stem cells in a cell sample.

Another object of the invention is a marker cocktail comprising one more marker conjugates each comprising at least one detection moiety and at least one antigen recognizing moiety against CD14, CD235a and CD15, wherein the ratio between the marker conjugates against CD14, CD235a and CD15 is 55-75%, 15 - 30% and 5 - 20% by weight.

Yet another object is a kit for detecting differentiated hematopoietic stem cells comprising cell media with at least one growth factor and a marker cocktail comprising one or more marker conjugates each comprising at least one detection moiety and at least one antigen recognizing moiety against CD14, CD235a and CD15 wherein the ratio between the marker conjugates against CD14, CD235a and CD15 is 55-75%, 15 - 30% and 5 - 20% by weight.

In the method of the invention, a cell media comprising a growth factor is utilized. Such cell media are known to a person skilled in the art and a typical composition is shown in the examples. Hereinafter, cell media comprising a growth factor are referred to as "HSC-CFU Assay Media" or "Assay Media". Such media are available from Miltenyi Biotec B.V. & Co. KG under the tradename "StemMACS HSC-CFU Assay Media".

The marker cocktail of the invention or for use in the invention is referred to as "Antibody Cocktail" or "HSC-CFU Antibody Cocktail", available available from Miltenyi Biotec B.V. & Co. KG under the tradename " StemMACS HSC-CFU Antibody Cocktail".

### BRIEF DESCRIPTION OF THE DRAWINGS

Fig. 1 shows a typical result of a CFC colony assay from a 35mm dish. The dots represent different types of colonies.
Fig. 2: The different type of colonies with their specific characteristics from the CFU assay in higher magnification.
Fig. 3: Results obtained after evaluation of the same 35mm dish from six independent researchers. The colonies were first enumerated and then listed sequentially so that each researcher can characterize each colony in terms of type. Fig. 3a shows the dish with the enumerated colonies. Fig. 3b shows the results obtained from the six independent researchers manifesting the high degree of variability observed after visual inspection. These findings strongly suggest that morphological and phenotypic classification is neither robust nor reproducible.
Fig. 4: Comparison of average total colony counts found in two 35cm wells and average total colony count in three 96-well plates when seeded with the same cell concentration. Error bars represent standard deviation.
Fig. 5: Gating strategy. Regions necessary for the analysis are highlighted.
Fig. 6: BFU-E characterization. A BFU-E colony is positive for the erythroid marker CD235a.
Fig. 7 shows CFU-G characterization.
Fig. 8 shows CFU-M characterization. A CFU-M colony should be at least 50% positive for CD14.
Fig. 9 shows CFU-GM characterization. A colony can be characterized as CFU-GM when it expresses more than 30% CD14 positive- and more than 30% CD15 positive cells. It should also be negative for the erythroid marker CD235a.
Fig. 10: CFU-GEMM characterization. The essence of CFU-GEMM is that is contains cells of all myeloid progenitors and is also positive for the expression of the erythroid marker CD235a.

### DETAILED DESCRIPTION

To address the issues of low reproducibility in the colony evaluation as well as to increase the robustness and eliminate the bias in the CFU assay, we developed the process according to the invention. The process according to the invention offers a highly standardized method for analyzing hematopoietic stem and progenitor cells because it combines differentiation in cell culture with a standardized, flow cytometry-based read-out and eliminates the need for user-dependent, visual scoring under a microscope.

The process of the invention allows detection or identification of colony forming cells and optionally the discrimination of colonies derived from those cells, especially hematopoietic stem cells (HSCs).

The undifferentiated or differentiated hematopoietic cells are preferable undifferentiated or differentiated hematopoietic stem cells.

In step a) of the method of the invention cells are isolated in groups of 1 - 1000 cells on a surface. Preferable, the number of cells is smaller like 100 to 500 cells or 150 to 350 cells.

For the colony formation, cells may be diluted in the methylcellulose-free HSC-CFU Assay Media and deposited for example into a 96-well plate at a concentration of 2.5 cells/well. In this way, each well corresponds to the clonal progeny of a single hematopoietic stem or progenitor cell. During the following incubation period (14 days), the HSC-CFU Assay Media promotes growth and differentiation of the deposited cells in suspension. Following their formation, the colonies are further assessed in terms of their type by staining each well of the 96-well plate with the HSC-CFU Antibody Cocktail and are subsequently analyzed by flow cytometry.

Thus, each colony can be easily identified through the corresponding marker combination. Moreover, based on the frequency of each colony type, the percentage of appearance of each colony can be determined versus the total number of colonies. This setup provides a standardized, user-independent analysis of HSC-CFU assays and allows for automation in combination with the any flow cytometric analyzer.

With the method of the invention, determination of the differentiation status of stem cells in a cell sample is possible. To this end, differentiated hematopoietic stem cells in a cell colony are detected as CFU-GEMM, CFU-GM, CFU-M, BFU-E and CFU-G by the relative amount of cells labelled with the marker conjugates as defined in the following table.

**Table 3 Colony detection parameters. The difference to 100% relates to other antigens which are not of interest for the method of the invention.**

| | Relative amount in % more than | | |
|---|---|---|---|
| | CD15+ | CD14+ | CD235a+ |
| CFU-GEMM | 15 | 15 | 20 |
| CFU-GM | 30 | 30 | 0 - 5 |
| CFU-M | 0 - 5 | 50 | 0 - 5 |
| BFU-E | 0 - 5 | 0 - 5 | 50 |
| CFU-G | 50 | 0 - 5 | 0 - 5 |

Preferable, the method according to the invention is performed in absence of methyl cellulose.

Depending on the origin of the cell sample, it is advisable to remove red blood cells and/or to enrich CD34+ cells before subjecting the cell sample to the method.

Removal of red blood cells may be performed by lysis or precipitation of the red blood cells from the sample. Such techniques are known to the person skilled in the art.

Enriching CD34+ cells from the cell sample comprising undifferentiated hematopoietic stem cells is preferable performed to a purity of at least 50%, more preferred to a purity of at least 90%. Enriching processes, for example by magnetic cell sorting or flow cytometric analyzer which are known to the person skilled in the art.

The detection moiety of the marker conjugates has no particular relevance for the method and may be selected from the group consisting of chromophore moiety, fluorescent moiety, phosphorescent moiety, luminescent moiety, light absorbing moiety, radioactive moiety, transition metal and isotope mass tag moiety. However, marker conjugates comprising one or more fluorescent moieties are preferred.

The same applies to the antigen recognizing moiety of the marker conjugate, which may be selected from the group consisting of antibody, an fragmented antibody, an fragmented antibody derivative, peptide/MHC-complexes targeting TCR molecules, cell adhesion receptor molecules, receptors for costimulatory molecules or artificial engineered binding molecules.

Concerning the marker cocktail and the kit of the invention, it is preferred that at least 3 different marker conjugates each comprising at least one antigen recognizing moiety against CD14, CD235a and CD15, respectively are provided, wherein the ratio between the marker conjugates against CD14, CD235a and CD15 is 55-75%, 15 - 30% and 5 - 20% by weight..

### EXAMPLES

### Materials and Methods

HSC-CFU Assay Media are media formulation that supports the growth of human BFU-E, CFU-E, CFU-G, CFU-M, CFU-GM and CFU-GEMM colonies. A typical composition is shown in table 1.

**Table 1**

| Components | Concentration in medium |
|---|---|
| Fetal bovine serum (FBS) | 30% |
| Bovine serum albumin (BSA) | 1% |
| L-glutamine | 2 mM |
| 2-mercaptoethanol | 0,1mM |
| Stem cell factor (SCF) | 50 ng/ml |
| GM-CSF | 20 ng/ml |
| G-CSF | 20 ng/ml |
| IL-3 | 20 ng/ml |
| IL-6 | 20 ng/ml |
| Erythropoietin (Epo) | 3 U/ml |

The following reagent and instruments are required, some are optional
- Flow cytometer with the ability to discriminate APC, PE, and VioBlue, e.g., MACSQuant Analyzer
- Accessories for processing 96-well plates at the flow cytometer, e.g. MACS^{®} Chill 96 Rack (# 130-094-459), when using the MACSQuant Analyzer 10
- Sterile 15 polypropylene tubes
- Sterile disposable pipette tips
- Sterile pipettes
- 96-well round bottom plates
- Humidity chamber
- Dilution medium: Iscoves's Modified Dulbecco's Medium (IMDM)
- PBS/EDTA buffer with 0,5% BSA (PEB), sterile and non-sterile
- Sterile water
- Multi-channel pipettor
- Reagent reservoirs
- CD34-APC
- CD45-FITC for optional measurement of white blood cells. A portion of the CD45 positive cells is also CD34 positive
- FCR blocking reagent
- (Optional) Red Blood Cell Lysis Solution (10x) (130-094183)

### Preparation of HSC-CFU Assay Media

To avoid repeated freeze-thaw cycles, the HSC-CFU Assay Media should be dispensed into appropriate aliquots. The protocol is as follows
1. Thaw the medium overnight at 4°C.
2. Shake the bottle vigorously.
3. Aliquot into sterile tubes (15.0 ml/tube) using a sterile pipette,
4. Freeze aliquots at -20°C. Thaw at room temperature before use or overnight at 4°C.

### Preparation of cell samples

Hematopoietic colony-forming assays can be performed using mononuclear cells from bone marrow, cord blood, or peripheral blood. Likewise, enriched hematopoietic stem and progenitor cells, e.g. enriched lineage marker-negative (Lin-), CD133+, or CD34+ cells, or ES and iPS cell-derived progenitors can be used.

For pre-enrichment of CD133+, CD34+, or Lin- cells, refer to the data sheet of the respective separation product.

### Set-up of HSC-CFU Assay :

The protocol is as follows
1. Thaw the required number of aliquoted HSC-CFU Assay Media at room temperature or overnight at 4°C. Each 15 mL aliquot corresponds to 1 test/assay and is sufficient for processing three 96-well plates. (Optional) For samples with high erythrocyte content, it is highly recommended to perform red blood cell lysis before determining the CD34+ cell count. Follow the instructions of the Red Blood Cell Lysis Solution (10x). Resuspend the cell pellet in sterile PEB.
2. Using sterile conditions, take a small sample up to 20µl and determine the cell number.
3. Remove an aliquot of up to 10E6 cells using sterile technique and proceed with steps 4-5.
4. Centrifuge at 300xg for 10 minutes. Aspirate supernatant completely. 5. Resuspend in 96 µL of non-sterile PEB.
6. Add 2 µL CD34-APC and 2 µL CD45-FITC.
7. Mix well and incubate for 10 minutes in the dark in the refrigerator (2-8°C).
8. Wash cells by adding 1-2 mL of buffer and centrifuge at 300xg for 10 minutes. Aspirate supernatant completely.
9. Resuspend the cell pellet in a suitable amount of non-sterile PEB for flow cytometric analysis Adjust the cell concentration to 250 CD34+CD45+ cells per 1 mL medium using plain IMDM.
10. Note: For each sample, three 96-well round-bottom plates are required. This 5 corresponds to 1000 CD34+ cells in 4 ml IMDM.
11. Vortex the tube to ensure even distribution of cells.
12. Transfer cell suspension into a reagent reservoir.
13. Pipette 10 µL into each well of three 96-well round bottom plates
14. Transfer 15 mL of HSC-CFU Assay medium into a new reagent reservoir.
15. Pipette 50 µL into each well of three 96-well round bottom plates
16. Place plates into a humidity chamber or sterile enclosure filled with a few mL of sterile water to minimize vaporization of cell culture medium during cultivation.
17. Incubate the plates for 12-14 days in a humidified incubator at 37°C and 5% CO₂.

### Flow cytometric analysis

The protocol is as follows
1. Prepare fluorochrome cocktail by diluting 45 µL of HSC-CFU Antibody Cocktail with PBS/EDTA/0,5% BSA buffer to a final volume of 4.5 mL.
2. Add 15 µL of diluted cocktail into each well of the three 96-well round bottom plates.
3. Add 25 µL of PBS/EDTA/0,5% BSA buffer to each well to a total volume of 100 µL
4. Proceed with acquisition.
5. Note: Make sure that the flow cytometer has been set up for processing of 96-well plates. A gentle mixing mode is recommended if supported by the flow cytometer.

### Set-Up of HSC-CFU Assay according to the prior art (comparative example)

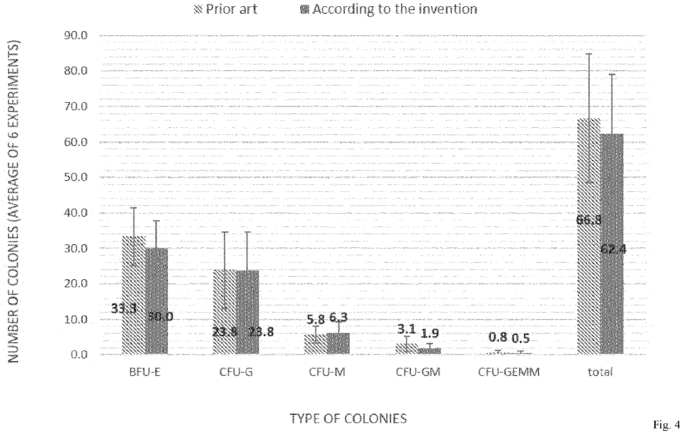

This assay is comparable with the classic methylcellulose CFU assay. In the latter, a total of 500-1000 CD34+ cells are plated in 3 ml of semisolid medium (or 250-500 CD34+ cells/ 1.5 ml) and then the medium is divided into two 35mm wells (~1 ml per well). From initial experimentation, we observed that the ideal number of plated CD34+ cells that gives the most reliable output in terms of colony size, colony type and total number of colonies/well was 250 cells/1ml. Based on that observation, and given that in the 96 well plate a total volume of 960µl is plated (96 wells x 10µl per well) and that not all CD34+ cells generate colonies, we performed experiments with different cell seeding numbers and we concluded that the ideal plating is 250 cells/960 µl, that corresponds to a concentration of 2.5 cells per well. Indeed, results obtained with this methodology generated comparable numbers of colonies between the semi-solid based CFU-assay ("classic" CFU-assay) in 35mm dishes and the liquid based CFU-assay in 96 well plates (Figure 4). Therefore the correlation is: 250 cells/35mmdish corresponds to ~ 250 cells/96 well plate. Per sample a total number of three 96 well plates are plated.

Figure 4 shows a comparison of average total colony counts found in two 35cm wells and average total colony count in three 96-well plates when seeded with the same cell concentration. Error bars represent standard deviation.

The cumulative results of a total of 6 experiments are also illustrated in Table 2 and showed that 96-well data is comparable to 35mm dish data. The p-value was higher than 0.05 (two tailed t-test) for all measurements, suggesting there is no statistical difference between the two assays in terms of colony count and colony type formation.

**Table 2 Cumulative data**

| Type | 35 mm dish | 96-well plate | p-valve |
|---|---|---|---|
| BFU-E | 33.3 | 30.0 | 0.27401122 |
| CFU-G | 23.8 | 23.8 | 0.98668047 |
| CFU-M | 5.8 | 6.3 | 0.60714795 |
| CFU-GM | 3.1 | 1.9 | 0.06952218 |
| CFU-GEMM | 0.8 | 0.5 | 0.28543638 |
| Total number of colonies | 66.8 | 62.4 | 0.50895526 |

### Immunofluorescent staining with the HSC-CFU Antibody Cocktail and subsequent data analysis

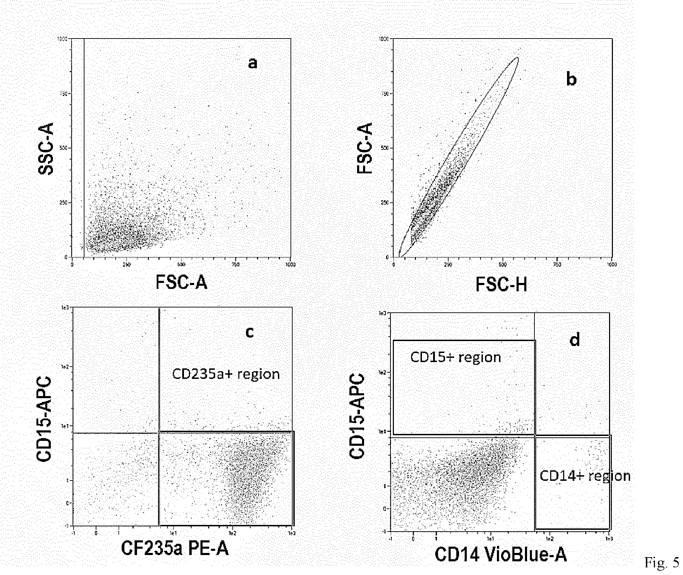

CD34+ cells from buffy coat were cultured in HSC-CFU Assay Medium for 14 days, stained with HSC-CFU Antibody Cocktail as described above and analyzed on a MACSQuant Analyzer 10. The process for analysis is described as per following instructions:
1. Perform an initial flow cytometric analysis be selecting a red colony (such as a BFU-E colony) with a positive signal in CD235a.
2. Draw a gate to include all events (Figure 5a).
3. Next, exclude all doublets by gating on single cells in a FSC-A vs. FSC-H plot. (Figure 5b).
4. Display all single cells in two new plots: For the first one (Figure 5c) adjust the y- axis to CD15-APC and x-axis to CD235a-PE. For the second one (Figure 5d), adjust the y-axis to CD15-APC and x-axis to CD14 VioBlue. Set a quadrant parting the populations as shown (Figures 5c and 5d).
5. Name the regions of interest: Add a name to the CD235a-PE positive region of plot c, the CD15-APC positive and CD14-VioBlue positive regions of plot d.
6. Apply this analysis template to all remaining wells. Information about the percentage of CD14+, CD15+, and CD235a+ cells in each quadrant is generated and exported into an excel worksheet for further computational analysis. Please, always make sure that the general gating parameters still apply, and that no regions have been shifted after re-compensation of the flow cytometer.

### Data analysis

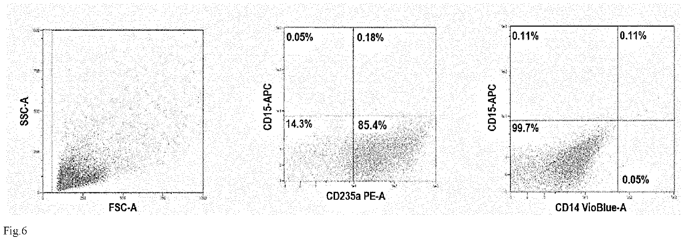

Flow cytometric analysis will return a specific staining for each well and therefore each well will display distinct positive events in one or more of the regions marked in Fig. 5 corresponding to respective markers. Based on these markers each colony can be classified by the percentage of stained cells in each of those regions. The colonies are characterized as BFU-E, CFU-GEMM, CFU-M and CFU-GM based on Figures 6- 10 and the guidelines summarized in Table 3.

BFU-E colonies can be determined by the number of positive CD235a events they exhibit. Over 50% of the total events have to be positive for CD235a-PE. Figure 6 shows BFU-E characterization. A BFU-E colony is positive for the erythroid marker CD235a.

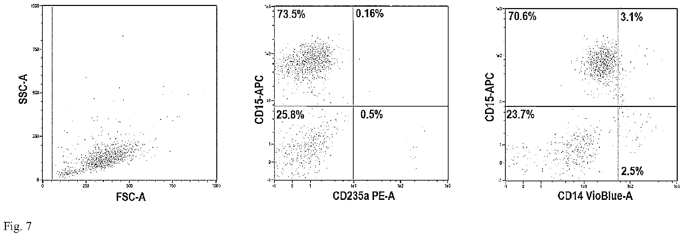

When over 50% of the total events are CD15-APC positive, the colony is a CFU-G. Figure 7 shows CFU-G characterization. A CFU-G colony is negative for the erythroid marker CD235a and expresses CD15 in more than 50% of the cells.

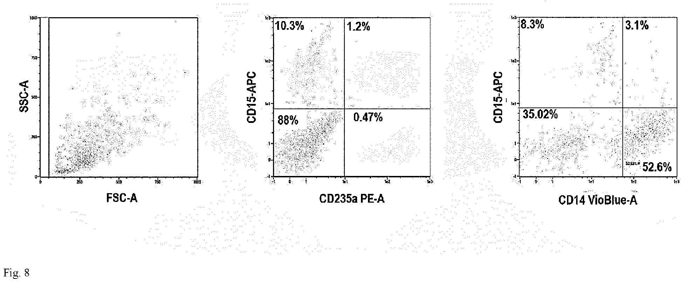

Cells belonging to a CFU-M colony are CD14 positive for over than 50% of the total events. Figure 8 shows CFU-M characterization. A CFU-M colony should be at least 50% positive for CD14.

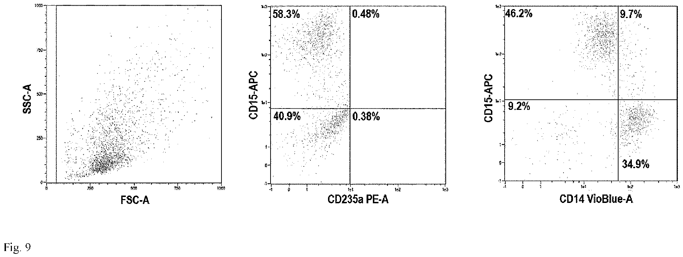

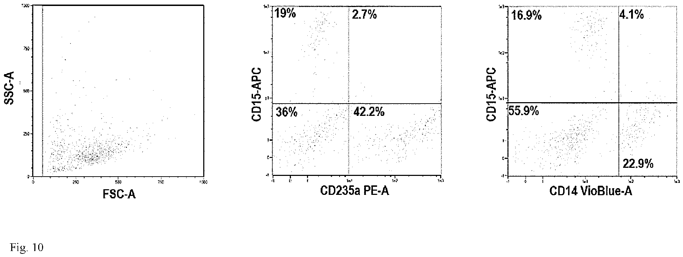

A CFU-GM colony contains progenitors of the granulocyte and monocyte/macrophage lineage. Therefore the staining in the CD14-VioBlue positive and CD15-APC positive region must be over 30% for each of those. The colony should be also negative for the erythroid marker CD235a. Figure 9 shows CFU-GM characterization. A colony can be characterized as CFU-GM when it expresses more than 30% CD14 positive- and more than 30% CD15 positive cells. It should also be negative for the erythroid marker CD235a. Figure 10 shows CFU-GEMM characterization. The essence of CFU-GEMM is that is contains cells of all myeloid progenitors and is also positive for the expression of the erythroid marker CD235a.

CFU-GEMM colonies are multilineage progenitors that contain cells of all other 5 myeloid progenitors. The criteria that have to be met to determine a CFU-GEMM are CD235a-PE positive events have to be over 20% of total cells, CD15-APC positive events have to be over 15% of total cells and CD14-VioBlue positive events have to be over 15% of total cells at the same time.

To summarize, based on the specific staining for each well the colonies can be 10 identified using the detections parameters listed on Table 3. For example, any well which exhibits at least 15% CD15, 14% CD14 and 20% CD235a positive events, would be a CFU-GEMM colony.

The hematopoietic system is constantly self-renewing and comprises cells at various stages of maturation. These include rare primitive stem cells with multi-lineage differentiation capacity and high self-renewal as well as progenitor cells with restricted differentiation and self-renewal potential. Today, semi-solid culture media have become the standard for enumeration and evaluation of stem and progenitor cells as colony forming-units (CFU). Based on methylcellulose in IMDM, supplemented with fetal bovine serum (FBS) and different growth factors, these media mimic the effect of stromal cells and provide optimal growth conditions. As previously mentioned, after the end of the incubation period the colonies are evaluated after visual observation under an optical microscope. However, the results obtained this way are biased and prone to error as they are totally dependent on the expertise of the researcher in charge of the assay. One way to simplify the analysis of CFU assays while still obtaining information about lineage-specific progenitor cell growth would be to perform assays in lineage-specific media in which all colonies are derived from one progenitor cell subtype, for example only CFU-GM or BFU-E, etc. However, cultures of this type do not represent the entire reservoir of progenitors/ stem cells and do not create the basis for robust evaluation. Thus, the only way to address the issues of low reproducibility in the visual colony evaluation as well as to increase the robustness and eliminate any bias, is to develop a method for systematically analyzing colonies, combining both differentiation in cell culture with a user-independent read-out assay.

To this end, we developed the process according to the invention which offers a standardized method for analyzing hematopoietic stem and progenitor cells because it combines differentiation in cell culture followed by flow cytometry-based evaluation and therefore, the need for user-dependent, visual scoring under a microscope is eliminated.

Following their formation, the colonies are analyzed by flow cytometry and are assessed in terms of their type after staining with the HSC-CFU Antibody Cocktail. Thus, each colony can be easily identified through the corresponding marker combination. Moreover, based on the frequency of each colony type, the percentage of appearance of each colony can be determined versus the total number of colonies. The innovation of the Assay and process according to the invention lies both on the methylcellulose-free HSC-CFU Medium as well as the simultaneous combination with the HSC-CFU Antibody Cocktail because it allows both for differentiation of the stem/progenitor cells as well as it provides a comprehensive characterization of each colony based on the specific staining it presents. This setup provides a standardized, user-independent analysis of HSC-CFU assays, allows for automation in combination with any flow cytometric analyzer and finally provides reproducible and consistent results.

## Claims

1. An *in vitro* method for detecting differentiated hematopoietic cells comprising the steps:
a) isolation of undifferentiated hematopoietic stem cells in groups of 1 - 1000 cells on a support
b) proliferating the isolated cells to form cell colonies of differentiated hematopoietic cells by providing cell media comprising a growth factor
c) contacting the cell colonies with one or more marker conjugates comprising at least one detection moiety and at least one antigen recognizing moiety against CD14, CD235a and CD15.
d) detecting the relative amount of differentiated hematopoietic stem cells in a cell colony labelled with the marker conjugates.

2. Method according to claim 1 **characterized in that** differentiated hematopoietic stem cells in a cell colony are detected as CFU-GEMM, CFU-GM, CFU-M, BFU-E and CFU-G by the relative amount of cells labelled with the marker conjugates
| | Relative amount in % more than | | |
|---|---|---|---|
| | CD15+ | CD14+ | CD235a+ |
| CFU-GEMM | 15 | 15 | 20 |
| CFU-GM | 30 | 30 | 0-5 |
| CFU-M | 0-5 | 50 | 0-5 |
| BFU-E | 0-5 | 0-5 | 50 |
| CFU-G | 50 | 0-5 | 0-5 |

3. Method according to claim 1 or 2 **characterized in that** the method is performed in absence of methyl cellulose.

4. Method according to any of the claims 1 to 3 **characterized in that** a cell sample comprising undifferentiated hematopoietic stem cells wherein red blood cell are lysed is provided to step a).

5. Method according to any of the claims 1 to 4 **characterized in that** a cell sample comprising undifferentiated hematopoietic stem cells wherein CD34+ cells are enriched is provided to step a).

6. Method according to claim 5 **characterized in that** the CD34+ cells of the cell sample are enriched to a purity of at least 50%.

7. Method according to any of the claims 1 to 6 **characterized in that** the detection moiety is selected from the group consisting of chromophore moiety, fluorescent moiety, phosphorescent moiety, luminescent moiety, light absorbing moiety, radioactive moiety, transition metal and isotope mass tag moiety.

8. Method according to any of the claims 1 to 7, wherein the antigen recognizing moiety is an antibody, an fragmented antibody, an fragmented antibody derivative, peptide/MHC-complexes targeting TCR molecules, cell adhesion receptor molecules, receptors for costimulatory molecules or artificial engineered binding molecules.

9. Use of the method according to at least one of the claims 1 to 8 to determining the differentiation status of stem cells in a cell sample.

10. Marker cocktail comprising one more marker conjugates each comprising at least one detection moiety and at least one antigen recognizing moiety against CD14, CD235a and CD15, wherein the ratio between the marker conjugates against CD14, CD235a and CD15 is 55-75%, 15 - 30% and 5 - 20% by weight.

11. Kit for detecting differentiated hematopoietic stem cells comprising cell media with at least one growth factor and a marker cocktail comprising one more marker conjugates each comprising at least one detection moiety and at least one antigen recognizing moiety against CD14, CD235a and CD15, wherein the ratio between the marker conjugates against CD14, CD235a and CD15 is 55-75%, 15 - 30% and 5 - 20% by weight..

## Patentansprüche

1. *In*-*vitro*-Verfahren zum Nachweisen differenzierter hämatopoetischer Zellen, das die folgenden Schritte umfasst:
a) Isolieren von undifferenzierten hämatopoetischen Stammzellen in Gruppen von 1 - 1.000 Zellen auf einem Träger,
b) Vermehren der isolierten Zellen zur Bildung von Zellkolonien aus differenzierten hämatopoetischen Zellen durch Bereitstellen von Zellmedien, die einen Wachstumsfaktor umfassen,
c) Inkontaktbringen der Zellkolonien mit einem oder mehreren Markerkonjugaten, die mindestens eine Nachweiskomponente und mindestens eine Antigenerkennungskomponente gegen CD14, CD235a und CD15 umfassen,
d) Nachweisen der relativen Menge an differenzierten hämatopoetischen Stammzellen in einer mit den Markerkonjugaten markierten Zellkolonie.

2. Verfahren nach Anspruch 1, **dadurch gekennzeichnet, dass** differenzierte hämatopoetische Stammzellen in einer Zellkolonie als CFU-GEMM, CFU-GM, CFU-M, BFU-E und CFU-G durch die relative Menge der mit den Markerkonjugaten markierten Zellen nachgewiesen werden.
| | Relative Menge in % größer | | |
|---|---|---|---|
| | CD15⁺ | CD14⁺ | CD235a⁺ |
| CFU-GEMM | 15 | 15 | 20 |
| CFU-GM | 30 | 30 | 0 - 5 |
| CFU-M | 0 - 5 | 50 | 0 - 5 |
| BFU-E | 0 - 5 | 0 - 5 | 50 |
| CFU-G | 50 | 0 - 5 | 0 - 5 |

3. Verfahren nach Anspruch 1 oder 2, **dadurch gekennzeichnet, dass** das Verfahren in Abwesenheit von Methylcellulose durchgeführt wird.

4. Verfahren nach einem der Ansprüche 1 bis 3, **dadurch gekennzeichnet, dass** eine Zellprobe, die undifferenzierte hämatopoetische Stammzellen umfasst, in denen rote Blutkörperchen lysiert sind, für Schritt a) bereitgestellt wird.

5. Verfahren nach einem der Ansprüche 1 bis 4, **dadurch gekennzeichnet, dass** eine Zellprobe, die undifferenzierte hämatopoetische Stammzellen umfasst, die mit CD34⁺-Zellen angereichert ist, für Schritt a) bereitgestellt wird.

6. Verfahren nach Anspruch 5, **dadurch gekennzeichnet, dass** die CD34⁺-Zellen der Zellprobe mit einer Reinheit von mindestens 50 % angereichert sind.

7. Verfahren nach einem der Ansprüche 1 bis 6, **dadurch gekennzeichnet, dass** die Nachweiskomponente ausgewählt ist aus der Gruppe, bestehend aus einer chromophoren Komponente, einer fluoreszierenden Komponente, einer phosphoreszierenden Komponente, einer lumineszierenden Komponente, einer lichtabsorbierenden Komponente, einer radioaktiven Komponente, einem Übergangsmetall und einer Komponente zur Isotopenmassenmarkierung.

8. Verfahren nach einem der Ansprüche 1 bis 7, wobei die Antigenerkennungskomponente Folgendes ist: ein Antikörper, ein fragmentierter Antikörper, ein fragmentiertes Antikörperderivat, Peptid/MHC-Komplexe, die auf TCR-Moleküle gerichtet sind, Zelladhäsionsrezeptormoleküle, Rezeptoren für kostimulatorische Moleküle oder synthetisch hergestellte Bindungsmoleküle.

9. Verwendung des Verfahrens nach mindestens einem der Ansprüche 1 bis 8 zur Bestimmung des Differenzierungsstatus von Stammzellen in einer Zellprobe.

10. Marker-Cocktail, bestehend aus einem oder mehreren Markerkonjugaten, die jeweils mindestens eine Nachweiskomponente und mindestens eine Antigenerkennungskomponente gegen CD14, CD235a und CD15 umfassen, wobei das Verhältnis zwischen den Markerkonjugaten gegen CD14, CD235a und CD15 55 - 75 Gew.-%, 15 - 30 Gew.-% und 5 - 20 Gew.-% beträgt.

11. Kit zum Nachweisen von differenzierten hämatopoetischen Stammzellen, die Zellmedien mit mindestens einem Wachstumsfaktor und einem Marker-Cocktail mit einem weiteren Markerkonjugat umfassen, die jeweils mindestens eine Nachweiskomponente und mindestens eine Antigenerkennungskomponente gegen CD14, CD235a und CD15 umfassen, wobei das Verhältnis zwischen den Markerkonjugaten gegen CD14, CD235a und CD15 55-75 Gew.-%, 15-30 Gew.-% und 5-20 Gew.-% beträgt.

## Revendications

1. Procédé *in vitro* de détection de cellules hématopoïétiques différenciées comprenant les étapes consistant à :
a) l'isolement de cellules souches hématopoïétiques indifférenciées en groupes de 1 à 1 000 cellules sur un support
b) faire proliférer les cellules isolées pour former des colonies cellulaires de cellules hématopoïétiques différenciées en fournissant des milieux cellulaires comprenant un facteur de croissance
c) mettre en contact les colonies cellulaires avec un ou plusieurs conjugués de marqueur comprenant au moins une fraction de détection et au moins un antigène reconnaissant une fraction contre CD14, CD235a et CD15
d) détecter la quantité relative de cellules souches hématopoïétiques différenciées dans une colonie cellulaire marquée avec les conjugués de marqueur.

2. Procédé selon la revendication 1 **caractérisé en ce que** les cellules souches hématopoïétiques différenciées dans une colonie cellulaire sont détectées comme CFU-GEMM, CFU-GM, CFU-M, BFU-E et CFU-G par la quantité relative de cellules marquées avec les conjugués de marqueur
| | Quantité relative en % de plus de | | |
|---|---|---|---|
| | CD15+ | CD14+ | CD235a+ |
| CFU-GEMM | 15 | 15 | 20 |
| CFU-GM | 30 | 30 | 0 - 5 |
| CFU-M | 0 - 5 | 50 | 0 - 5 |
| BFU-E | 0 - 5 | 0 - 5 | 50 |
| CFU-G | 50 | 0 - 5 | 0 - 5 |

3. Procédé selon la revendication 1 ou 2 **caractérisé en ce que** le procédé est effectué en absence de méthyl cellulose.

4. Procédé selon l'une quelconque des revendications 1 à 3 **caractérisé en ce qu'**un échantillon cellulaire comprenant des cellules souches hématopoïétiques indifférenciées les globules rouges étant lysés est fourni à l'étape a).

5. Procédé selon l'une quelconque des revendications 1 à 4 **caractérisé en ce qu'**un échantillon cellulaire comprenant des cellules souches hématopoïétiques indifférenciées les cellules CD34+ étant enrichies est fourni à l'étape a).

6. Procédé selon la revendication 5 **caractérisé en ce que** les cellules CD34+ de l'échantillon cellulaire sont enrichies à une pureté d'au moins 50 %.

7. Procédé selon l'une quelconque des revendications 1 à 6 **caractérisé en ce que** la fraction de détection est choisie dans le groupe constitué par une fraction de chromophore, une fraction fluorescente, une fraction phosphorescente, une fraction luminescente, une fraction absorbant la lumière, une fraction radioactive, un métal de transition et une fraction d'étiquette de masse d'isotope.

8. Procédé selon l'une quelconque des revendications 1 à 7, dans lequel la fraction reconnaissant l'antigène est un anticorps, un anticorps fragmenté, un dérivé d'anticorps fragmenté, des complexes peptide/MHC ciblant des molécules TCR, des molécules de récepteur d'adhésion cellulaire, des récepteurs pour molécules costimulatoires ou des molécules de liaison modifiées artificielles.

9. Utilisation du procédé selon l'une quelconque des revendications 1 à 8 pour déterminer le statut de différenciation de cellules souches dans un échantillon cellulaire.

10. Cocktail de marqueurs comprenant un ou plusieurs conjugués de marqueur chacun comprenant au moins une fraction de détection et au moins un antigène reconnaissant la fraction contre CD14, CD235a et CD15, le rapport entre les conjugués de marqueur contre CD14, CD235a et CD15 étant de 55 à 75 %, 15 à 30 % et 5 à 20 % en poids.

11. Kit pour détecter les cellule souches hématopoïétiques différenciées comprenant des milieux cellulaires avec au moins un facteur de croissance et un cocktail de marqueurs comprenant un ou plusieurs conjugués de marqueur chacun comprenant au moins une fraction de détection et une fraction reconnaissant un antigène contre CD14, CD235a et CD15, le rapport entre les conjugués de marqueur contre CD14, CD235a et CD15 étant de 55 à 75 %, 15 à 30 % et 5 à 20 % en poids.
